# EUROPÄISCHE PATENTSCHRIFT

(11) **EP 1 562 638 B1**
(45) Veröffentlichungstag und Bekanntmachung des Hinweises auf die Patenterteilung: **11.01.2012**
(21) Anmeldenummer: 03782209.5
(22) Anmeldetag: 18.11.2003
(51) Int. Cl.: A61K 49/00

(54) **LYMPHKNOTEN-KONTRASTMITTEL**
LYMPH NODE CONTRAST AGENT
PRODUIT DE CONTRASTE DE GANGLION LYMPHATIQUE

(30) Priorität: 18.11.2002 EP 02025600
(43) Veröffentlichungstag der Anmeldung: 17.08.2005
(73) Patentinhaber: Till, Uwe Prof. Dr., 99087 Erfurt (DE)
(72) Erfinder: Till, Uwe Prof. Dr., 99087 Erfurt (DE)
(74) Vertreter: HOFFMANN EITLE
(86) Internationale Anmeldenummer: PCT/EP2003/012891
(87) Internationale Veröffentlichungsnummer: WO 2004/045650

(56) Entgegenhaltungen:
- WO-A-00/35490
- WO-A-94/21303
- WO-A-2004/080491
- US-A- 4 187 285
- US-A- 5 114 703
- US-A- 6 159 445
- US-B1- 6 205 352
- TASDEMIROGLU EROL ET AL: "Effect of superoxide dismutase on acute reperfusion injury of the rabbit brain." ACTA NEUROCHIRURGICA, Bd. 120, Nr. 3-4, 1993, Seiten 180-186, XP009008134 ISSN: 0001-6268
- NICOLAYSEN G ET AL: "Time course of albumin equilibration in interstitium and lymph of normal mouse lungs." MICROVASCULAR RESEARCH. UNITED STATES JAN 1975, Bd. 9, Nr. 1, Januar 1975 (1975-01), Seiten 29-37, XP009008107 ISSN: 0026-2862
- MOLECULAR PROBES, 'FLUOSPHERES FLUORESCENT MICROSPHERES', PRODUCT INFORMATION, [Online] XP002276334 Gefunden im Internet: <URL:http://www.interchim.fr/bio/molprobes /cd/docs/media/pis/mp05000.pdf> [gefunden am 2004-04-06]
- HANATANI K ET AL: "Molecular weight-dependent lymphatic transfer of fluorescein isothiocyanate-labeled dextrans after intrapulmonary administration and effects of various absorption enhancers on the lymphatic transfer of drugs in rats" JOURNAL OF DRUG TARGETING 1995 UNITED KINGDOM, Bd. 3, Nr. 4, 1995, Seiten 263-271, XP009029088 ISSN: 1061-186X
- HERAUSGEGEBEN VON J. FALBE, M. REGITZ: RÖMPP CHEMIE LEXIKON, 10. AUFLAGE, 1997, pages 1576-1577, Stuttgart

## Beschreibung

Die Erfindung betrifft ein Kontrastmittel auf Farbstoffbasis für die Lymphknoten-Darstellung, und zwar insbesondere die Darstellung von Sentinel-Lymphknoten. Solche Kontrastmittel dienen beispielsweise einem Chirurgen während einer Tumoroperation als wichtiges Hilfsmittel für die Erkennung und anschließende Entfernung eines Lymphknotens; Ferner betrifft die Erfindung Verfahren zur Herstellung solcher Kontrastmittel und deren Verwendung als Diagnostika in der Medizin.

### Hintergrund der Erfindung

Die häufigsten bösartigen Tumoren des Menschen setzen ihre Tochtergeschwülste primär in dem zugehörigen Lymphabflussgebiet ab. Auf diese Weise entstehen Lymphknotenmetastasen. Bei der operativen Tumorentfernung werden daher in der Regel die Tumor-benachbarten Lymphknotenstationen so komplett wie möglich entfernt. Nachfolgend werden die entfernten Lymphknoten in der Pathologie auf Metastasen untersucht. Von dem Ergebnis hängt die weitere Behandlung ab. Neben dem operativen Aufwand und der großen Zahl histomorphologischer Untersuchungen kann dieses Verfahren vor allem nach der Operation mit einer erheblichen gesundheitlichen Beeinträchtigung der Patienten verbunden sein, wie etwa durch Behinderung des Lymphabstroms, Narbenbildung, Nervenschädigung usw.

Nach dem sogenannten Sentinel(=wächter)-Lymphknoten-Konzept wird bei der Bildung von Tochtergeschwülsten primär immer der Lymphknoten befallen, der als erster im Abstrombereich des Tumors stationiert ist (Büchels HK, Vogt H, Wagner T, Steinfeld D, Sagassser J, Sentinel-Lymphonodektomie beim Mammakarzinom, Der Nuklearmediziner 1999, 22; 261-267). Bei Beschränkung auf die Entfernung dieses Knotens (oder dieser Knoten bei mehreren Abstromgebieten) und den während der Operation durch sogenannten Schnellschnitt geführten Nachweis einer Metastasenfreiheit dieses Knotens könnte die Operation in diesem Stadium beendet werden. Der operative und histomorphologische Aufwand und die Beeinträchtigung der Patienten wären somit weitaus geringer. Dieses "Sentinel-Lymphknotenprinzip" ist derzeit für die operative Entfernung von Melanomen weitgehend etabliert und befindet sich für viele andere Turmorarten in klinischer Erprobung (Vogt H, Wengenmair H, Kopp J, Dorn R, Gröber S, Heidenreich P, Der Sentinel-Lymphknoten (SLN): prä- und intraoperative nuklearmedizinische Diagnostik, Der Nuklearmediziner 1999, 22, 233-242; Balch CM, Lange JR, Lymphatic Mapping and Sentinel Node Lymphadenectomy for Cancer: An Overview, Annals of Surgical Oncology 2001, 8(9S), 1-4; Zervos EE, Burak WE, Lymphatic Mapping in Solid Neoplasms: State of the Art, Cancer Control 2002, (8)3, 189-202).

Zur Erfassung von Sentinel-Lymphknoten werden derzeit vor allem zwei methodische Prinzipien eingesetzt:

Zum einen werden in Lösung befindliche kolloidale Partikel durch Technetium-99m radioaktiv markiert und vor der Operation in die unmittelbare Umgebung des Tumors injiziert. Die Partikel werden dann über die Lymphwege abtransportiert und in dem bzw. den Sentinel-Lymphknoten gespeichert. Ihr Nachweis erfolgt meist in zwei Schritten: a) am Vortag der Operation durch Nachweis der Radioaktivität mittels Ganzkörperszintigrafie und b) während der Operation mit eigens dafür konstruierten Gamma-Sonden als Radioaktivitätsdetektoren. Die Nachteile dieser Methode sind allerdings beträchtlich (siehe Heidenreich P, Vogt H, Bachter D, Büchels H, Steinfeld D, Wawroschek F, Wengenmair H, Wagner T, Das Konzept des Wächterlymphknotens, Dt. Ärzteblatt 2001, 98; A534-540):
- Sie ist an das Vorhandensein einer nuklearmedizinischen Abteilung gebunden.
- Sie ist technisch außerordentlich aufwändig. Neben der Ganzkörperszintigrafie sind speziell konstruierte Gamma-Sonden notwendig, die je nach Operationsgebiet verschieden sein müssen.
- Sie ist mit einer Strahlenexposition von Operationsteam, Patienten und Pathologen verbunden und erfordert entsprechende Vorkehrungen.
- Die eindeutige Lokalisation des Sentinel-Lymphknotens während der Operation ist mitunter nicht möglich, wegen zu enger Verhältnisse oder wenn der Sentinel-Lymphknoten sehr nahe dem Injektionsort liegt, an dem die meiste Radioaktivität liegen bleibt und so den Lymphknoten überstrahlt.

Trotz dieser Nachteile wird - in Ermangelung besserer Alternativen - diese Methode gegenwärtig in vielen Krankenhäusern angewendet.

Zum anderen werden für die Lymphknotendarstellung biokompatible, blaue Farbstoffe in die unmittelbare Umgebung des Tumors kurz vor der Operation injiziert. Dadurch färben sich Lymphbahnen und -knoten. Aber auch diese Methode hat ihre Nachteile (Bachter D, Starz H, Volkmar C, Vogt H, Büchels H, Balda BR, Die Sentinel-Lymphonodektomie beim malignen Melanom, Der Nuklearmediziner 1999, 22; 245-252):
- Die gegenwärtig eingesetzten Farbstoffe färben zwar zunächst die Sentinel-Lymphknoten, werden dort aber nicht komplett zurückgehalten, sondern passieren sie. Sie färben daher später auch andere, entfernter liegende Lymphknoten, so dass der Sentinel-Lymphknoten nicht immer eindeutig festgestellt werden kann. Dadurch ist der Operateur unter Umständen gezwungen, unnötig viele Lymphknoten zu entfernen, was mit einer entsprechenden post-operativen Belastung für den Patienten verbunden ist.
- Die Farbstoffe gehen in das Blut über und werden über Galle und Urin ausgeschieden. Es kommt zur Blaufärbung der Patienten, die einige Tage anhalten kann.

Die Nachteile der beiden Verfahrensweisen können teilweise durch die Kombination beider Techniken vermindert werden, indem z.B. eine grobe Orientierung durch die Gamma-Sonde durch die Färbung der Lymphknoten verbessert wird (Zervos EE, Burak WE, Lymphatic Mapping in Solid Neoplasms: State of the Art, Cancer Control 2002, (8)3, 189-202). Mit einer solchen Kombination wurden bislang die besten Ergebnisse erreicht (Allan R, Sentinel node localization: do or dye alone? Br J Radiol 2001, 74; 475-477). Der notwendige apparative und präparative Aufwand ist aber dann sehr hoch.

WO 00/35490 beschreibt Kontrastmittel auf Grundlage eines Konjugats aus einem Makromolekül wie z.B. Dextran und einem Farbstoff wie z.B. einem Triazin-Farbstoff zur Verwendung in der Lymphknotendiagnostik.

US 6,205,352 beschreibt eine nicht-radioaktive Zusammensetzung zur Identifikation von Sentinel-Lymphknoten mit freiem Auge, die aus Partikeln wie z.B. Mikrospheren oder Liposomen bestehen, die einen Farbstoff tragen.

### Zusammenfassung der Erfindung

Ausgehend von diesem Stand der Technik ist es das der Erfindung zugrundeliegende Ziel, eine Zusammensetzung für ein Verfahren der Lymphknotendarstellung bereitzustellen, das nicht die Nachteile der oben diskutierten, bekannten Verfahren aufweist. Insbesondere soll ein ebenso zuverlässiges und weniger aufwändiges Verfahren bereitgestellt werden als das bisher meistens eingesetzte radiologische Sentinel-Lymphknoten-Diagnostizierverfahren.

Ferner soll erfindungsgemäß eine Zusammensetzung bereitgestellt werden, die sich für ein solches Verfahren einsetzen lässt.

Dieses Ziel wird erfindungsgemäß erreicht durch Bereitstellung der Verwendung [eines nicht-radiomarkiertes Kontrastmittels enthaltenal physiologisch verträglichen und pharmazeutisch unbedenkliche Dextran-Partikeln,die ein Molekulargewicht von 1-5 Millionen aufweisen und zu 80% (bezogen auf die Teilchenzahl) oder mehr eine Teilchengröße im Bereich von 30 bis 300 nm aufweisen und eine Farbe haben, die sich mit bloßem Auge von der Farbe menschlichen Gewebes unterscheiden lässt, zur Darstellung des oder der Sentinel-Lymphknoten eines Tumorpatienten.

Die Erfindung stellt auch einen Kit bereit, umfassend einen ersten Behälter mit dem nicht-radiomarkierten Kontrastmittel, umfassend als wesentlichen Bestandteil physiologisch verträgliche und pharmazeutisch unbedenkliche Dextran-Partikel, die ein Molekulargewicht von 1-5 Millionen aufweisen und zu 80 % oder mehr eine Teilchengröße im Bereich von 30 bis 300 nm aufweisen und eine Farbe haben, die sich mit bloßem Auge von der Farbe menschlichen Gewebes unterscheiden lässt, und einen zweiten Behälter umfassend eine Zinn(II)chlorid-Lösung, sowie ein radioaktiv-markiertes Kontrastmittel, welches aus dem erfindungsgemäßen Kit nach Vermischen der Kitkomponenten durch Neutralisieren und Umsetzen der so erhaltenen Mischung mit ^{99m}Tc-pertechnat erhältlich ist.

Vorzugsweise sind die Teilchen mit einem pharmazeutisch akzeptablen Farbstoff angefärbt, der entweder im Bereich des sichtbaren Lichts oder im UV wenigstens ein Wellenlängenabsorptions- oder Emissionsmaximum hat, das bei entsprechender Beleuchtung seine Unterscheidung von menschlichem Gewebe mit bloßem Auge erlaubt. Besonders bevorzugt haben die angefärbten Partikel bzw. der Farbstoff, mit dem sie angefärbt wurden, ein Absorptionsmaximum im Bereich von 550 bis 680 nm oder ein Emissionsmaximum im Bereich von 380 bis 780 nm nach Anregung im UV/VIS-Bereich.

### Partikel

Die erfindungsgemäß verwendeten Dextran-Partikel weisen, bezogen auf die Partikelanzahl, zu 80 % oder mehr, vorzugsweise zu über 90 %, besonders bevorzugt zu 95 % oder mehr, eine Teilchengröße im Bereich von 30 bis 300 nm auf. Partikel in diesem Größenbereich werden zwar durch die Lymphkanäle transportiert, bleiben aber in den Lymphknoten hängen.

Die erfindungsgemäß verwendeten Partikel können (näherungsweise) globuläre oder nicht globuläre Partikel sein.

Für die Partikel ist die Einstellung und Bestimmung der Partikelgröße durch fraktionierte Filtration durch Membranfilter zu empfehlen. Bei Verwendung von Filtern des MF-Typs der Firma Millipore kommen z.B. solche mit den Porengrößen 25, 50, 100, 220, 300, 450, 650, 800 und 1.200 nm in Frage. Bei Kohlenhydratpartikeln, wie z.B. Dextranpartikeln, wird die Teilchengröße durch Gelchromatographie als sogenannter Stokes-Durchmesser erhalten.

Die erfindungsgemäß verwendeten Partikel müssen ferner für den Patienten physiologisch verträglich und pharmazeutisch unbedenklich sein, weil sie in vivo verabreicht werden. Die Kriterien für physiologische Verträglichkeit und pharmazeutische Unbedenklichkeit ergeben sich aus den Anforderungen der Arzneimittelbehörden; für die Zwecke dieser Erfindung kann davon ausgegangen werden, dass ein Kontrastmittel bzw. die darin enthaltenen Bestandteile dann physiologisch verträglich und pharmazeutisch unbedenklich ist bzw. sind, wenn das Kontrastmittel von der EMEA oder einer nationalen Gesundheitsbehörde (z.B. FDA in den USA, oder BfArM in Deutschland) für die in vivo Diagnostik von Lymphknoten zugelassen wurde. Die physiologische Verträglichkeit und pharmazeutische Unbedenklichkeit schließt ein, dass das Kontrastmittel in steriler, pyrogenfreier und virenfreier Form vorliegt. Ferner sollten die Partikel nicht immunogen sein und keine toxischen Metaboliten bilden.

Beispiele für Partikel, die solchen Anforderungen genügen, sind dem Fachmann bekannt und werden teilweise bereits in anderen in vivo-Applikationen eingesetzt. Besonders geeignet für solche Partikel sind Dextrane, Stärken, unsubstituierte oder substituierte Zellulosederivate, z.B. Acacia, Chitosane, Pectine, Carrageenane, oder Alginate, soweit biokompatibel und in den gewünschten Partikelgrößen erhältlich oder herstellbar.

Bei der Herstellung der erfindungsgemäßen Partikel kann die Teilchengröße durch eine Ultraschallbehandlung, eine fraktionierte Fällung, ein Molsiebverfahren, Filtrationstechniken oder Zentrifugieren kontrolliert und eingestellt werden.

Geeignet sind Dextranpartikel entsprechender Größe. Das primäre Produkt wird durch den Lactobazillenstamm *Leuconostoc mesenteroides B512* synthetisiert und deshalb biologisch aus dessen Kulturmedium gewonnen. Es besteht aus α-(1→6)-verknüpften D-Glucopyranosemolekülen, die über α-(1→3) -Bindungen verzweigt sind (J.W. van Cleve, W.C. Schäfer, C.E. Rist, The structure of NRRLB-512 dextran. Methylation studies, J. Amer. Chem. Soc. 78 (1956) 4435-4438 und S. Lindberg, S. Svenson, Structural studies on dextran from Leuconostoc mesenteroides NRRL B-512. Acta Chem. Scand. 22 (1968) 1907-1912). Das aus dem Kulturmedium isolierte Dextran ist ein Gemisch aus sehr verschiedenen Molekulargewichten. Durch partielle Hydrolyse können sehr große Ketten verkürzt und über mehrfache Fraktionierung mittels Ethanol/Wasser-Gemischen die gesuchten Partikelgrößen isoliert werden (B. Ingelman, M.S. Halling, Some physicochemical experiments on fractions of dextran. Ark. Kemi 1 (1949) 61-80).

Erfindungqgemäβ verwendet werden Dextranfraktionen mit Molekulargewichten von 1-5 Millionen. Verfügbar ist Dextran T-2000 (Hersteller: Amersham Biosciences). Das mittlere Molekulargewicht dieses Produkts beträgt ca. 2 Millionen. Es hat eine fadenförmige bis gestreckt-knäuelige Struktur und einen Stokes-Durchmesser, bestimmt durch Gelchromatographie, von etwa 54 nm (Amersham Biosciences, User Report of DEXTRAN T-2000).

### Farbstoff

Der Farbstoff zur Verwendung im erfindungsgemäßen Kontrastmittel sollte pharmazeutisch akzeptabel und als Stoff für die in vivo Verabreichung am Menschen zugelassen sein. Seine Funktion ist die der farblichen Markierung des Sentinel-Lymphknotens für den operierenden Arzt, ohne dass dieser zur Erkennung des Sentinel-Lymphknotens auf aufwändige Technologien, z.B. Gammasonden, zurückgreifen muss. Vielmehr sollte der Operateur den bzw. die Sentinel-Lymphknoten mit bloßem Auge ohne weiteres erkennen können. Entsprechend sollte der Farbstoff entweder im Bereich des sichtbaren Lichts oder im UV wenigstens ein Wellenlängenabsorptions-oder Emissionsmaximum haben, das bei entsprechender Beleuchtung (entweder mit normalem Licht oder UV-Licht) seine Unterscheidung von menschlichem Gewebe mit bloßem Auge erlaubt. Vorzugsweise hat der Farbstoff ein Absorptionsmaximum im Bereich von 550 bis 680 nm und/oder ein Emissionsmaximum im Bereich von 380 bis 780 nm nach Anregung im UV/VIS-Bereich. Unter den Farbstoffen mit einem Emissionsmaximum im Bereich von 380 bis 780 nm nach Anregung im UV/VIS-Bereich sind unter anderem Fluoreszenzfarbstoffe bevorzugt, insbesondere solche, die im sichtbaren Bereich nach Anregung im UV emittieren und somit ohne Filter verwendet werden können. Besonders bevorzugt sind dunkelblaue oder intensiv grüne Farbstoffe. Schwarze Farbstoffe kommen ebenfalls in Frage.

Farbstoffe aus zahlreichen Farbstoffklassen eignen sich zum Einsatz in den erfindungsgemäßen Kontrastmitteln. Besonders bevorzugt sind Azofarbstoffe, Bisazofarbstoffe, Trisazofarbstoffe, Diarylmethan-, Triarylmethanfarbstoffe, Anthrachinonfarbstoffe, polyzyklische aromatische Carbonylfarbstoffe, Indigofarbstoffe, Phthalocyanine, Nitrosofarbstoffe, Triphenodioxazinfarbstoffe, FormazanFarbstoffe (Formazan-Kupfer-Komplexe) undAza-Annulene.

Ganz besonders bevorzugt sind konkret z.B. die Farbstoffe Acid blue 40, Acid green 27, Amido black, Chicago sky blue, Cibacron blue F3G-A, F-R, FN-R, P-3R, Cibacron brillant blue H-GR, Cibacron Black BG, Lansol Black B (die Cibacron- und Lanasol-Farbstoffe sind erhältlich von Ciba Speciality Chemicals Inc.), Evans-Blue, Fluoresceine (Fluorescein und Carboxyfluoresceine und ihre Derivate, wie z.B. 2', 7'-Difluorfluorescein, die als Reaktivfarbstoffe in Form ihrer Isothiocyanate oder Succinimidylester verwendet werden können), Indigocarmin, Indigotetrasulfonat, Isosulfan blue., Methylenblau, Monastral blue, Patentblau V, Brilliant Blau FCF, Grün S (E142), Levafix Brilliant Blue EFFA, Levafix Black EB, Levafix Black E-2G, Levafix Black P-36A, Levafix Black PN-L (die Levafix-Farbstoffe sind von Bayer erhältich) , Procion Blue MX-R, Procion Blue MX-4GD, Procion Blue MX-G, Procion Blue MX-2GN (die Procion-Farbstoffe sind von ICI erhältlich), Dye 555 ((Hersteller: Dyomics), Sumifix Black B und Sumifix Black H-BG (erhältlich von Sumitomo Chemical Company), Lucifer Yellow, Rhodamine und Trypanblau.

Die Anfärbung der erfindungsgemäßen Partikel durch den Farbstoff kann durch chemische und/oder physikalische Wechselwirkungen (Chemisorption oder Physisorption) vermittelt werden. Unter dem Gesichtspunkt der Stabilität sind kovalente chemische Bindungen zwischen Partikel und Farbstoff bevorzugt, die durch Reaktion mit einem Reaktivfarbstoff erhalten werden können. Aber auch physikalische Wechselwirkungen, z.B. hydrophobe Wechselwirkungen, Wasserstoffbrücken-Bildung u.ä. können für eine zufriedenstellende Anfärbung der Partikel völlig ausreichen, solange der Farbstoff-Partikel-Komplex auch in vivo hinreichend stabil bleibt. Bei Kohlenhydratpartikeln ist eine kovalente Farbstoffanbindung besonders bevorzugt. Ein praktisches Maß für im Sinne der Erfindung ausreichende Stabilität bzw. Bindungsfestigkeit von Farbstoff an Partikel ist beispielsweise, dass in der für die Applikation vorgesehenen Flüssigkeit in 24 Stunden bei 37°C nicht mehr als maximal 5 Gew.-% des ursprünglich gebundenen Farbstoffs von den Partikeln abgelöst (freigesetzt) werden.

Im Sinne der Erfindung sind Partikel, die durch einen Farbstoff angefärbt sind, auch Partikel, die im Molekül selbst Chromophore enthalten und daher eine Eigenfarbe aufweisen.

Für die Zwecke dieser Erfindung geeignete Bisazofarbstoffe sind substituierte Aromaten mit zwei Azogruppen, die im sichtbaren Bereich ein Absorptionsmaximum im Bereich von Wellenlängen zwischen etwa 550 und 680 nm haben. Farbstoffe mit einer solchen Absorptionswellenlänge sind in der Regel blau oder grün und somit für die Anwendung bei der Lymphknotendarstellung besonders kontrastreich. Die Extinktion des Farbstoffs bei der maximalen Absorptionswellenlänge sollte vorzugsweise so hoch sein, dass man den Farbstoff mit bloßem Auge im Gewebe gut erkennen kann, wenn er in der zur Injektion üblichen Menge appliziert wird.

Bisazofarostoffe eignen sich.in besonderem Maße für die Anfärbung von Partikeln.

Evans blue(4,4'-Bis(1-amino-8-hydroxy-2,4-disulfo-7-naphthylazo)-3,3'-bitolyl-Tetranatriumsalz) ist aufgrund seiner physiologischen Verwendbarkeit (siehe z.B. El-Sayed et al., Re-evaluation of Evans blue dye dilution method of plasma volume measurement, Clin.Lab.Haem. 1995, 17, 189-194) in besonderer Weise als Farbstoff in den erfindungsgemäßen Kontrastmitteln geeignet. Evans blue wurde bereits in ungebundener Form für die Lymphknotendarstellung verwendet (siehe J.-Y. Bobin et al., Tagging Sentinel Lymph Nodes: a Study of 100 Patients with Breast Cancer, Eur.J.Cancer, Ausgabe.35, Nr.4, 569-573, 1999). Die Verwendung von Evans blue für die Lymphknotendarstellung würde jedoch später als nachteilig beschrieben, insbesondere gegenüber Patentblau, und daher hat sich in der Praxis letzteres durchgesetzt (siehe z.B. Bachter et al., Die Sentinel-Lymphonodektomie beim malignen Melanom, Der Nuklearmediziner, 22, V245-252 1999). Bisher wurden sowohl Patentblau als auch Evans Blue allerdings immer in ungebundener Form direkt verwendet, während ihre Verwendung zum Anfärben von Partikeln und der Einsatz solcher Partikel für die Lymphknoten-Darstellung nicht beschrieben oder vorgeschlagen wurde.

Unter den Farbstoffen mit einem Absorptionsmaximum im Wellenlängenbereich von 550 bis 680 nm ist der Farbstoff Cibacron F-R, gekoppelt an Kohlenhydrate wie z.B. Dextrane, aufgrund der starken Färbung wie auch der Festigkeit der Bindung besonders geeignet.

Unter den Fluoreszenzfarbstoffen eignet sich besonders Fluorescein (als Fluoresceinisothiocyanat (FITC) umgesetzt), insbesondere gekoppelt an Kohlenhydratpartikel wie z.B. Dextranpartikel Die Verwendung eines Dextran-FITC-Konjugates, z.B. eines Dextran T-2000-FITC Konjugates, bei dem 0,01 bis 0,02 mol FITC pro Glucoseeinheit gebunden sind, ermöglicht bei Abdunkelung und Bestrahlung mit einer UV-Lampe eine besonders empfindliche Detektion, bei der der Sentinel-Lymphknoten zitronengelb erscheint.

### Herstellung des endgültigen Kontrastmittels

Nach der Herstellung von geeigneten angefärbten Partikeln können diese mit weiteren Formulierungshilfsmitteln (Additiven) versetzt und anschließend für die Lagerung konserviert werden, z.B. durch Sterilisierung und gegebenenfalls Lyophilisierung, so dass man - ggf. nach Auflösung in physiologischer Kochsalzlösung oder einem anderen geeigneten Lösungsmittel - ein anwendungsfertiges Kontrastmittel erhält.

Das erfindungsgemäß herzustellende Kontrastmittel zur Detektion von Sentinel-Lymphknoten enthält die erfindungsgemäßen angefärbten Partikel vorzugsweise in steriler und pyrogenfreier Form enthält. Die Formulierung sollte aus medizinischen und zulassungsrechtlichen Gründen insbesondere frei von Hepatitis B-Oberflächenantigenen (HbsAg), Antikörpern für das humane Immundefizienz-Virus (anti-HIV 1/2) sowie Antikörpern für den Hepatitis C-Virus (anti-HCV) sein. Die Formulierung kann in Form einer anwendungsfertigen physiologisch akzeptablen Lösung, die vorzugsweise isotonisch sein sollte, bereitgestellt werden, oder in lyophilisierter Form.

Beispielsweise kann eine geeignete Formulierung Glucose, ein nichtionisches Tensid, das die Agglomeration verhindert, wie z.B. Pluronic (z.B. Pluronic F-68), Phosphatpuffer (Natriumphosphat) und/oder Natriumphytat enthalten. In der einfachsten Variante enthalten die erfindungsgemäßen Kontrastmittel jedoch lediglich die erfindungsgemäß angefärbten Partikel in einem geeigneten isotonischen Puffersystem, z.B. Phosphatpuffer pH 7.4 oder Trispuffer pH 8.0, oder in isotonischer Kochsalzlösung.

Das nicht-radioaktiv-markierte Kontrastmittel kann zur Herstellung eines radioaktiv-markierten Kontrastmittels verwendet werden, die aufgrund der kurzen Halbwertszeit gängiger radioaktiver Marker wie ^{99m}Technetium erst kurz vor der Operation in der Klinik, z.B. durch einen Radiologen, erfolgt. Um die Herstellung im Klinikbereich zu vereinfachen, stellt die Erfindung auch ein Kit zur Herstellung von entsprechend radioaktiv-markierten Kontrastmitteln aus den erfindungsgemäßen nicht-radioaktiv-markierten Kontrastmitteln bereit, das sich zusammensetzt aus dem erfindungsgemäßen nicht-radioaktiv-markierten Kontrastmittel und, räumlich getrennt hiervon, einer Zinn(II)-Lösung in Salzsäure. Die beiden Bestandteile des Kits können z.B. in getrennten Behältern in Mengen abgefüllt sein, dass der Radiologe die jeweiligen Mengen lediglich miteinander vermischen und anschließend das Gemisch neutralisieren muss, bevor er es mit z.B. ^{99m}Tc-pertechnate umsetzt, um so ein 99m-markiertes Kontrastmittel zu erhalten.

### Anwendung der Kontrastmittel in der Tumorbehandlung

Die Erfindung betrifft die Verwendung der wie oben beschrieben angefärbten Partikel zur Herstellung eines nicht-radiomarkierten Kontrastmittels zur Darstellung des oder der Sentinel-Lymphknoten eines Tumorpatienten. Die Kontrastmittel kommen vorzugsweise zum Einsatz während der operativen Entfernung eines Tumors, insbesondere eines Melanoms, Mammakarzinoms, Zervixkarzinoms oder Prostatakarzinoms.

Diese Verwendung ist insbesondere dann vorteilhaft, wenn die Lymphabflussrichtung des Tumors bekannt ist. Das ist - abhängig von der Art und der Lokalisierung des Tumors - oft der Fall. Die Kontrastmittel können unmittelbar vor der Operation (abhängig von Art und Sitz des Tumors in der Regel zwischen 10 min und 5 h vorher) injiziert werden, so dass der bzw. die Sentinel-Lymphknoten während der Operation durch ihre Anfärbung mit den erfindungsgemäßen Partikeln mit bloßem Auge erkannt werden können. Der gesamte technische, finanzielle und personelle Aufwand der Radionuklidtechnik kann dann entfallen. Auch die Applikation am Vortag der Operation ist möglich, wenn z.B. gleichzeitig 99m Technetiummarkierte Partikel gegeben werden, um mittels Scintigraphie die Lymphabflussrichtung zu ermitteln.

Bei dieser Verwendung wird einem Patienten vor der Operation eine erfindungsgemäß zu verwendende pharmazeutische Zusammensetzung mit angefärbten Partikeln als Kontrastmittel durch Injektion in den Tumor oder in seine unmittelbare Umgebung verabreicht. Dann wird eine ausreichende Zeit zugewartet, damit die angefärbten Partikel den Sentinel-Lymphknoten sicher erreichen. Abhängig von Art und Sitz des Tumors liegt dies zwischen 10 min und 2 h. Während der Operation wird dann der Tumor zusammen mit dem oder den angefärbten Sentinel-Lymphknoten entfernt. Je nach dem Zustand der Sentinel-Lymphknoten kommt dann die operative Entfernung weiterer Lymphknoten in Betracht. Wenn der Sentinel-Lymphknoten metastasenfrei ist, ist es nicht erforderlich, noch weitere Lymphknoten zu entfernen.

Wenn die Abflussrichtungen des Tumors nicht sicher bekannt sind, ist dagegen die Verwendung eines radioaktiv markierten Kontrastmittels, das aus dem erfindungsgemäßen Kit enthaltend das nicht-radiomarkierte Kontrastmittel erhältlich ist, vorteilhaft, weil sicherer. Über das radioaktive Isotop werden vor der Operation durch Ganzkörperszintigrafie die Abflussrichtungen ermittelt. Da der bzw. die Sentinel-Lymphknoten gleichzeitig durch die erfindungsgemäßen Partikel angefärbt ist bzw. sind, sind die Knoten intraoperativ leicht auffindbar. Es entfällt somit in dieser Variante die zweite Injektion freier Farbstoffe und der Einsatz von Gamma-Sonden, deren Konstruktion, Anpassung und rechnergestützte Auswertung zur Ausschaltung der Hintergrundstrahlung oder Steigerung der Auflösung außerordentlich aufwändig ist (siehe Heidenreich et al, Das Konzept des Wächterlymphknotens, Dt.Ärzteblatt 2001, 98, A534-540).

Die Erfindung wird durch die nachstehenden Beispiele erläutert.

### Beispiele

### Beispiel 1: (Referewobeispiel)

Herstellung einer erfindungsgemäßen Albuminpartikel-Zusammensetzung - ausgehend von Albu-Res (Nycomed Amersham Sorin)
1. 2,5 mg Albumin als Mikrokolloid aus einer Flasche Albu-Res werden in 1 ml Phosphatpuffer (8,2 mM Na₂HPO₄, 1,8 mM KH₂PO₄, 137 mM NaCl, pH 7,2) suspendiert.
2. 0,5 ml dieser Suspension werden mit 2 mg Evans blue (Merck) versetzt, gemischt und 15 min bei Raumtemperatur stehen gelassen.
3. Danach wird die Suspension auf eine Säule (d = 1 cm, h = 2,5 cm) aufgetragen, die mit Sephadex G-50 fine (Pharmacia) gefüllt ist, das mit dem Phosphatpuffer der o.g. Zusammensetzung äquilibriert ist.
4. Elution mit dem gleichen Phosphatpuffer und Fraktionierung zu je 1 ml. Der Evans blue/Albu-Res-Komplex erscheint im Außenvolumen.
5. Die Gipfelfraktionen (Nr. 2 bis 5) werden vereint und mittels Vakuum-Zentrifugalverdampfer auf 0,4 bis 0,5 ml konzentriert.

### Beispiel 2 (Referenzbeispiel)

Herstellung einer Dextranpartikel-Zusammensetzung - ausgehend von Dextran mit einem Molekulargewicht von 5-40 Millionen
1. 1 g Dextran (Molekulargewicht 5-40 Millionen, Hersteller: ICN Biomedicals) werden mit 20 ml bidestilliertem Wasser versetzt und unter Rühren bei Raumtemperatur ca. eine Stunde stehen gelassen.
2. Behandlung mit Ultraschall unter Kühlung bis die Suspension klar ist.
3. Zusatz von 100 mg des Trisazofarbstoffes CIBACRON Blau P-3R (Hersteller: Ciba) in 5 ml bidestilliertem Wasser gelöst und 15 min bei Raumtemperatur rühren.
4. Zusatz von 0,5 g NaCl und 30 min rühren.
5. Zusatz von 0,5 ml einer 5N NaOH, rühren und 20 Stunden bei Raumtemperatur stehen lassen.
6. Neutralisieren mit 5N HCl
7. Zentrifugieren (3.000xg), Überstand verwerfen und Niederschlag in 25 ml bidestilliertem Wasser resuspensieren.
8. Wiederholung von Schritt 7. bis der Überstand keine nennenswerte Blaufärbung mehr zeigt und Aufnahme des Niederschlages in 20 ml physiologischer Kochsalzlösung.

### Beispiel 3

Herstellung einer erfindungsgemäßen Dextranpartikel-Zusammensetzung - ausgehend von Dextran mit einem Molekulargewicht von etwa 2 Millionen
1. 1 g Dextran T-2000 (Molekulargewicht ca. 2 Millionen, Hersteller: Amersham) wird mit 20 ml bidestilliertem Wasser versetzt und unter Rühren bei Raumtemperatur, ca. eine Stunde stehen gelassen.
2. Zusatz von 100 mg des Trisazofarbstoffes CIBACRON Brillant Blau H-GR (Hersteller: Ciba) in 5 ml bidestilliertem Wasser gelöst und 15 min bei Raumtemperatur rühren.
3. Zusatz von 0,5 g NaCl und 30 min rühren.
4. Zusatz von 0,5 ml einer 5N NaOH, rühren und 20 Stunden bei Raumtemperatur stehen lassen.
5. Neutralisieren mit 5N HCl
6. Filtration durch ein Membranfilter mit einer Porenweite von 25 nm und Nachwaschen mit bidestilliertem Wasser, bis der Durchlauf frei von Farbstoff ist.
7. Auffüllen des Filtrationsrückstandes mit physiologischer Kochsalzlösung auf 20 ml.

### Beispiel 4 :

Herstellung einer erfindungsgemäßen Dextranpartikel-Zusammensetzung - ausgehend von Dextran mit einem Molekulargewicht von etwa 2 Millionen
1. 200 mg Dextran T-2000 (Molekulargewicht ca. 2 Millionen; Hersteller: Amersham) wurden mit 3 ml bidestilliertem Wasser versetzt und unter Rühren bei Raumtemperatur ca. eine Stunde stehen gelassen.
2. 150 mg des Formazanfarbstoffs Cibacron Blau F-R (Hersteller: Ciba) wurden zugesetzt und zum Lösen 15 min bei Raumtemperatur gerührt.
3. 80 µl einer 6 N NaOH wurden zugesetzt.
4. Die Lösung wurde 18 Stunden bei 40°C mit einer Frequenz von 120 min⁻¹ geschüttelt.
5. Nach Abkühlung wurden 5 ml Ethanol (99,9 %) zugesetzt, die Mischung 10 mal geschwenkt und anschließend ca. 30 sec im Vortex-Mischer geschüttelt. Nach 15 min Stehenlassen bei Raumtemperatur wurde der Überstand vorsichtig abgegossen. Nach erneutem Stehenlassen wurde der Rest des Überstands abgegossen.
6. 3 ml einer 0,16 N NaOH wurden zum Niederschlag zugegeben und 35 min gerührt.
7. Die Schritte 5. und 6. wurden 2 mal wiederholt und anschließend nochmals Schritt 5. durchgeführt.
8. 1,8 ml bidestilliertem Wasser wurden zugegeben und geschüttelt, bis der Niederschlag gelöst war (ca. 50 min Schütteln und 5 min im Vortex-Mischer)
9. 75 µl einer 18 %igen NaCl-Lösung wurden zugesetzt.
10. 100 µl Tris/HCl (1M, pH 8,0) wurden zugesetzt.

### Beispiel 5:

Beispiel 4 wurde wiederholt, wobei der Farbstoff Cibacron Blau F3GA anstelle des Farbstoffs Cibacron Blau F-R verwendet wurde.

### Vergleichsbeispiel 1:

Beispiel 4 wurde wiederholt, wobei Dextran mit einem Molekulargewicht von ca. 500.000 anstelle des Dextrans mit einem Molekulargewicht von ca. 2 Millionen verwendet wurde.

### Vergleichsbeispiel 2:

Beispiel 5 wurde wiederholt, wobei Dextran mit einem Molekulargewicht von ca. 500.000 anstelle des Dextrans mit einem Molekulargewicht von ca. 2 Millionen verwendet wurde.

### Beispiel 6 (Vergleichsbeispiel)

### Tierexperimentelle Validierung des erfindungsgemäße Kontrastmittels

Dieses Beispiel dient zur experimentellen Validierung der Brauchbarkeit einer gegebenen Partikel-Farbstoff-Kombination durch Vergleich der durch Anfärbung erhaltenen Lymphknoten-Markierung mit der nach einem radiologischen Standardverfahren erhaltenen radioaktiven Markierung. Obwohl in diesem Beispiel speziell die Kombination Albumin (Albu-res)-Evans Blue eingesetzt wurde, lassen sich völlig analog beliebige weitere Farbstoff-Partikel-Kombinationen experimentell validieren. So zeigen die Dextran/Cibacronblau-Komplexe gemäß den Beispielen 2 und 3 die gleichen Markierungsmuster wie 99m Technetium oder die in diesem Beispiel verwendeten Evans blue-markierten Albures-Partikel.

versuchstiere sind weibliche Wistar-Ratten (Alter: 3 monate, Gewicht 220 -270 g) .
1. Die Narkose wird mit Ether eingeleitet. Dann erhält das Tier Ketamin+Rompun (Mischungsverhältnis 1+1; 1 µl/g Körpergewicht) intramuskulär injiziert. Ketamin 100 mg/ml (Atarost); Rompun: 2 % (Bayer Vital)
2. In die rechte Vorder- und Hinterpfote der Tiere werden je 0,1 ml der gemäß Beispiel 1 hergestellten Zusammensetzung, der das zur Sentinel-Lymphknoten-Darstellung in klinischem Einsatz befindliche ^{99m}Technetium-markierte Albu-Res in einer Dosis von 1 MBq beigemischt ist, subkutan in den Ballen injiziert. Gleichzeitig werden in die linke Vorder- und Hinterpfote der Tiere je 0,1 ml der letztgenannten Zusammensetzung, ebenfalls in einer Dosis von 1 MBq, injiziert.
3. Nach einer Verteilungszeit von 60 min wird das Tier mit einer Überdosis an Ether getötet.
4. Die Lymphknoten beider Seiten werden frei präpariert, entfernt und entsprechend der Nummerierung in der Abbildung FIG.J-1 in: Hebel H, Stromberg MW [eds.] Anatomy and Embryology of the Laboratory Rat, BioMed Verlag Wörthsee, 1986, mit 1 bis 19b beziffert und geordnet.
5. Die Blaufärbung der Lymphknoten wird mit dem bloßen Auge durch den Beobachter bewertet (0: keine Färbung; 1: schwache Färbung; 2: starke Färbung) und die aufgenommene Radioaktivitätsmenge in einem Gamma-Counter gemessen.

Das Ergebnis eines repräsentative Versuches ist in den Tabellen 1 und 2 dokumentiert, bezüglich der Radioaktivität (RA) einmal als Absolutwerte (Tabelle 1) und zum anderen in Prozent der gesamten gemessenen Radioaktivität (Tabelle 2). Daraus ergibt sich, dass sich Radioaktivität und Blaufärbung nach gleichem Muster verteilen und daher mit dem Partikel/Farbstoff-Komplex die Sentinel-Lymphknoten genau so sicher erfasst werden wie mit dem radiologischen Verfahren. Dies trifft ausnahmslos auf alle bisher dazu durchgeführten Tierversuche zu. Auch Partikel/Farbstoff-Komplexe, die nach in den Beispielen 2 bis 4 aufgeführten Verfahren hergestellt wurden, zeigen das gleiche Verteilungsmuster wie der Albu-Res/Evans blue-Komplex.

**Tabelle 1**

| | | | |
|---|---|---|---|
| Versuche mit ^{99m}Tc- und Evans-Blue-markiertem Albures rechts: Evansblue/Albures + ^{99m}Tc/Albures; links: nur ^{99m}Tc/Albures | | | |

| **Tierseite** | **rechts** | | **links** |
|---|---|---|---|
| **LK** | **RA (cpm)** | **Färbung** | **RA (cpm)** |
| 1 | 68,2 | 0 | 127,4 |
| 2 | 23,9 | 0 | 31,0 |
| 3 | 8,7 | 0 | 20,5 |
| 4 | - | - | - |
| **5a** | **1.132.749,4** | **2** | **420.716,7** |
| 5b | 97.785,7 | 1 | 108.669,6 |
| 5c | 48.775,6 | 1 | 291.815,6 |
| 6a | 405.990,1 | 2 | 270.372,0 |
| 6b | 74.718,8 | 0 | 1.116,1 |
| 6c | 5.325,6 | 0 | 1.290,7 |
| 7 | 10,0 | 0 | 19,4 |
| 8 | - | - | - |
| 9 | 55,7 | 0 | 58,3 |
| 10 | 38,8 | 0 | 29,1 |
| 11 | - | - | - |
| 12 | 157,6 | 0 | 48,2 |
| 13* | 335,5 | 0 | 335,5 |
| 14 | 11.185,3 | 0 | 11.967,2 |
| 15 | - | - | - |
| 16* | 3.190,4 | 0 | 3.190,4 |
| 17 | - | - | - |
| 18 | 168.591,6 | 1 | 76.596,4 |
| 19a | 256,7 | 0 | 387,7 |
| 19b | 332,6 | 0 | 363,8 |
| **Summe (cpm)** | **1.949.600,2** | | **1.187.155,6** |

| | | | |
|---|---|---|---|
| LK=Lymphknoten; RA=Radioaktivität; cpm=counts per minute LK 13 und 16 liegen medial und sind singulär (RA auf beiden Seiten eingetragen) | | | |

**Tabelle 2**

| | | | |
|---|---|---|---|
| Versuche mit ^{99m}Tc- und Evans-Blue-markiertem Albures rechts: Evansblue/Albures + ^{99m}Tc/Albures; links: nur ^{99m}Tc/Albures | | | |

| **Tierseite** | **rechts** | | **links** |
|---|---|---|---|
| **LK** | **RA (%)** | **Färbung** | **RA (%)** |
| 1 | 0,003% | 0 | 0,011% |
| 2 | 0,001% | 0 | 0,003% |
| 3 | 0,000% | 0 | 0,002% |
| 4 | - | - | - |
| **5°** | **58,102%** | **2** | **35,439%** |
| 5b | 5,016% | 1 | 9,154% |
| 5c | 2,502% | 1 | 24,581% |
| 6a | 20,824% | 2 | 22,775% |
| 6b | 3,833% | 0 | 0,094% |
| 6c | 0,273% | 0 | 0,109% |
| 7 | 0,001% | 0 | 0,002% |
| 8 | - | - | - |
| 9 | 0,003% | 0 | 0,005% |
| 10 | 0,002% | 0 | 0,002% |
| 11 | - | - | - |
| 12 | 0,008% | 0 | 0,004% |
| 13* | 0,017% | 0 | 0,028% |
| 14 | 0,574% | 0 | 1,008% |
| 15 | - | - | - |
| 16* | 0,164% | 0 | 0,269% |
| 17 | - | - | - |
| 18 | 8,647% | 1 | 6,452% |
| 19a | 0,013% | 0 | 0,033% |
| 19b | 0,017% | 0 | 0,031% |
| **Summe (cpm)** | **100%** | | **100%** |

### Beispiel 7

### Vergleich verschiedener Dextran-Farbstoff-Konjugate im Tierexperiment

Versuchstiere wurden wie in Beispiel 6 beschrieben präpariert. Anschließend wurde den Tieren entsprechend der Vorgehensweise von Beispiel 6 jeweils die Kontrastmittel der Beispiele 4 und 5 sowie der Vergleichsbeispiele 1 und 2 verabreicht. Die Lymphknotenfärbungen, der Übergang ins Blut (Magen-Darm-Färbung) sowie der Anteil des Farbstoffs im Filtrat nach der Filtration mit einem 50 nm - Filter wurden untersucht. Die Ergebnisse sind in der folgenden Tabelle gezeigt:

| **Dextran (MG)** | **Cibacron-Blau** | **Färbung des Sentinel-LK** | **Färbung der LK der 2. Reihe** | **Übergang in das Blut** | **Anteil des Farbstoffs im Filtrat (50 nm - Filter)** |
|---|---|---|---|---|---|
| 0,5 Mio. | F3GA | Ja | Ja | Ja | 81,2 % |
| 0,5 Mio. | F-R | Ja | Ja | Nein | 9,1 % |
| 2 Mio. | F3GA | Ja | Nein | Ja | 56,3 % |
| 2 Mio. | F-R | Ja | Nein | Nein | 13,5 % |

| | | | | | |
|---|---|---|---|---|---|
| LK=Lymphknoten | | | | | |

Die Ergebnisse zeigen, dass ein Dextran-Farbstoff-Konjugat mit einem Molekulargewicht von 2 Mio. im Hinblick auf die fehlende Anfärbung der 2. Lymphknotenreihe zu einem eindeutigeren diagnostischen Ergebnis führt. Zudem wird durch die Verwendung des Farbstoffs Cibacron Blau F-R nicht nur eine deutliche Färbung des Sentinel-Lymphknotens erhalten, sondern auch ein Übergang des Farbstoffs ins Blut und damit eine unnötige Farbstoffexposition des Patienten vermieden.

## Patentansprüche

1. Verwendung eines nicht-radiomarkierten kontrastmittels enthaltend physiologisch verträgliche und pharmazeutisch unbedenkliche Dextranpartikel die ein Molekulargewicht von 1 bis 5 Millionen aufweisen und zu 80 % oder mehr, bezogen auf die Partikelanzahl, eine Teilchengröße im Bereich von 30 bis 300 nm aufweisen und eine Farbe haben, die sich mit bloßem Auge von der Farbe menschlichen Gewebes unterscheiden lässt, zur Darstellung des oder der Sentinel-Lymphknoten eines Tumorpatienten.

2. Verwendung nach Anspruch 1, **dadurch gekennzeichnet, dass** die Partikel mit einem pharmazeutisch akzeptablen Farbstoff angefärbt sind.

3. Verwendung nach Anspruch 2, **dadurch gekennzeichnet, dass** der Farbstoff ein Absorptionsmaximum im Bereich von 550 bis 680 nm oder ein Emissionsmaximum im Bereich von 380 bis 780 nm nach Anregung im UV/VIS-Bereich hat.

4. Verwendung nach einem der vorhergehenden Ansprüche, **dadurch gekennzeichnet, dass** die Partikel zu 90% oder mehr eine Teilchengröße im Bereich von 30 bis 300 nm aufweisen.

5. Verwendung nach einem der vorhergehenden Ansprüche, **dadurch gekennzeichnet, dass** die Anfärbung der Partikel durch die Farbstoffe über hydrophobe, ionische oder kovalente Wechselwirkungen oder über Komplexbildung derart erfolgt, dass die Partikel nicht mehr als maximal 5 Gew.-% des ursprünglich gebundenen Farbstoffes in dem zur Applikation vorgesehenen Lösungsmittel in 24 Stunden bei 37°C freisetzen.

6. Verwendung nach einem der vorhergehenden Ansprüche, **dadurch gekennzeichnet, dass** der Farbstoff ausgewählt ist aus Azofarbstoffen, Bisazofarbstoffen, Triazofarbstoffen, Diarylmethan-, Triarylmethanfarbstoffen, Antrachinonfarbstoffen, Pthalocyaninen, polycyclischen aromatischen Carbonylfarbstoffen, Triphenodioxazinfarbstoffen, Nitrosofarbstoffen, Indigofarbstoffen, Formazan-Farbstoffen (Formazan-Kupfer-Komplexe) und Aza-Annulenen.

7. Verwendung nach Anspruch 6, **dadurch gekennzeichnet, dass** der Farbstoff ausgewählt ist aus Acid blue 40, Acid green 27, Amido black, Chicago sky blue, Cibacron blue F3G-A, Cibacron blue F-R, Cibacron blue FN-R, Cibacron blue P-3R, Cibacron brillant blue H-GR, Evans-Blue, Fluoresceine, Indigocarmin, Indigotetrasulfonat, Isosulfan blue, Methylenblau, Monastral blue, Patentblau V, Rhodamine und Trypanblau.

8. Verwendung nach einem der vorhergehenden Ansprüche, **dadurch gekennzeichnet, dass** der Farbstoff ein Fluoreszenzfarbstoff ist, der bei Bestrahlung mit UV oder VIS-Licht ein Licht im Bereich von 380-780 nm Wellenlänge emittiert.

9. Verwendung nach einem der vorhergehenden Ansprüche, **dadurch gekennzeichnet, dass** das Kontrastmittel in steriler, pyrogenfreier und lyophilisierter Form vorliegt.

10. Verwendung nach einem der Ansprüche 1 bis 8, **dadurch gekennzeichnet, dass** das Kontrastmittel als Partikelsuspension in einem sterilen, pyrogenfreien, pharmazeutisch akzeptablen wässrigen Lösungsmittel vorliegt.

11. Verwendung gemäß einem der vorhergehenden Ansprüche, zur Darstellung des oder der Sentinel-Lymphknoten bei einem Melanom, Mamma-, Zervix- oder Prostatakarzinom.

12. Kit, umfassend einen ersten Behälter mit dem nicht-radiomarkierten Kontrastmittel, umfassend als wesentlichen Bestandteil physiologisch verträgliche und pharmazeutisch unbedenkliche Dextran-Partikel, die ein Molekulargewicht von 1 bis 5 Millionen aufweisen und zu 80 % oder mehr, bezogen auf die Partikelanzahl, eine Teilchengröße im Bereich von 30 bis 300 nm aufweisen und eine Farbe haben, die sich mit bloßem Auge von der Farbe menschlichen Gewebes unterscheiden lässt, und einen zweiten Behälter umfassend eine Zinn(II)chlorid-Lösung.

13. Radioaktiv-markiertes Kontrastmittel, erhältlich durch Vermischen eines nicht-radiomarkierten Kontrastmittels, umfassend als wesentlichen Bestandteil physiologisch verträgliche und pharmazeutisch unbedenkliche Dextran-Partikel, die ein Molekulargewicht von 1 bis 5 Millionen aufweisen und zu 80 % oder mehr, bezogen auf die Partikelanzahl, eine Teilchengröße im Bereich von 30 bis 300 nm aufweisen und eine Farbe haben, die sich mit bloßem Auge von der Farbe menschlichen Gewebes unterscheiden lässt, mit einer Zinn(II)chlorid-Lösung, Neutralisieren und Umsetzen mit ^{99m}Tc-Pertechnat.

## Claims

1. Use of a non-radiolabelled contrast agent containing physiologically compatible and pharmaceutically harmless dextran particles which have a molecular weight of 1 to 5 million and 80 % or more of which, relative to the particle number, have a particle size in the range from 30 to 300 nm and have a colour which can be distinguished with the naked eye from the colour of human tissue, for showing the sentinel lymph node or nodes of a tumour patient.

2. Use according to claim 1, **characterised in that** the particles are stained using a pharmaceutically acceptable dyestuff.

3. Use according to claim 2, **characterised in that** the dyestuff has an absorption maximum in the range from 550 to 680 nm or an emission maximum in the range from 380 to 780 nm after excitation in the UV/VIS range.

4. Use according to one of the preceding claims, **characterised in that** 90 % or more of the particles have a particle size in the range from 30 to 300 nm.

5. Use according to one of the preceding claims, **characterised in that** the staining of the particles by the dyestuffs is effected via hydrophobic, ionic or covalent interactions or via complex formation such that the particles release no more than 5 wt.% at the most of the originally bound dyestuff in the solvent provided for administration in 24 hours at 37°C.

6. Use according to one of the preceding claims, **characterised in that** the dyestuff is selected from azo dyestuffs, bisazo dyestuffs, triazo dyestuffs, diaryl methane dyestuffs, triaryl methane dyestuffs, anthraquinone dyestuffs, phthalocyanines, polycyclic aromatic carbonyl dyestuffs, triphenodioxazine dyestuffs, nitroso dyestuffs, indigo dyestuffs, formazan dyestuffs (formazan-copper complexes) and aza-annulenes.

7. Use according to claim 6, **characterised in that** the dyestuff is selected from Acid blue 40, Acid green 27, Amido black, Chicago sky blue, Cibacron blue F3G-A, Cibacron blue F-R, Cibacron blue FN-R, Cibacron blue P-3R, Cibacron brilliant blue H-GR, Evans Blue, fluoresceins, indigo carmine, indigo tetrasulphonate, isosulphane blue, methylene blue, Monastral blue, patent blue V, rhodamine and trypan blue.

8. Use according to one of the preceding claims, **characterised in that** the dyestuff is a fluorescent dyestuff which emits a light in the range from 380-780 nm wavelength on irradiation using UV or VIS light.

9. Use according to one of the preceding claims, **characterised in that** the contrast agent is present in sterile, pyrogen-free and lyophilised form.

10. Use according to one of claims 1 to 8, **characterised in that** the contrast agent is present as a particle suspension in a sterile, pyrogen-free, pharmaceutically acceptable aqueous solvent.

11. Use according to one of the preceding claims, for showing the sentinel lymph node or nodes in a melanoma, carcinoma of the breast, cervix or prostate.

12. Kit comprising a first container with the non-radiolabelled contrast agent, comprising as essential constituent, physiologically compatible and pharmaceutically harmless dextran particles which have a molecular weight of 1 to 5 million and 80 % or more of which, relative to the particle number, have a particle size in the range from 30 to 300 nm and have a colour which can be distinguished with the naked eye from the colour of human tissue, and a second container comprising a tin(II) chloride solution.

13. Radioactively labelled contrast agent which can be obtained by mixing a non-radiolabelled contrast agent, comprising as essential constituent physiologically compatible and pharmaceutically harmless dextran particles which have a molecular weight of 1 to 5 million and 80 % or more of which. relative to the particle number, have a particle size in the range from 30 to 300 nm and have a colour which can be distinguished with the naked eye from the colour of human tissue, with a tin(II) chloride solution, neutralising and reacting with ^{99m}Tc pertechnate.

## Revendications

1. Utilisation d'un produit de contraste non radiomarqué, contenant des particules de dextrane physiologiquement compatibles et pharmaceutiquement sans risque, présentant un poids moléculaire dans la fourchette de 1 à 5 millions et, pour jusqu'à 80 % ou plus, rapporté au nombre de particules, une grosseur de particule dans la fourchette de 30 à 300 nm, et ayant une couleur se distinguant à l'oeil nu de la couleur des tissus humains, pour visualiser un ou plusieurs ganglions lymphatiques sentinelles d'un patient touché par une tumeur.

2. Utilisation selon la revendication 1, **caractérisée en ce que** les particules sont colorées avec un colorant pharmaceutiquement acceptable.

3. Utilisation selon la revendication 2, **caractérisée en ce que** le colorant présente un maximum d'absorption dans la fourchette de 550 à 680 nm, ou un maximum d'émission dans la fourchette de 380 à 780 nm après excitation dans la plage UV/VIS.

4. Utilisation selon l'une des revendications précédentes, **caractérisée en ce que** les particules présentent, pour jusqu'à 90 % ou plus, une grosseur de particule dans la fourchette de 30 à 300 nm.

5. Utilisation selon l'une des revendications précédentes, **caractérisée en ce que** la coloration des particules est effectuée au moyen des colorants, par des interactions hydrophobes, ioniques ou covalentes, ou par formation de complexes, de manière que les particules ne dégagent pas plus d'un maximum de 5 % en poids du colorant initialement lié dans le solvant prévu pour l'application, en 24 heures, à 37°C.

6. Utilisation selon l'une des revendications précédentes, **caractérisée en ce que** le colorant est sélectionné parmi les colorants azo, colorants biazo, colorants triazo, colorants diarylméthane, triarylméthane, colorants antrachinons, phtalocyanines, colorants carbonylés aromatiques polycycliques, colorants triphénodioxazine, colorants nitroso, colorants indigo, colorants formazane (complexes formazane-cuivre) et aza-annulènes.

7. Utilisation selon la revendication 6, **caractérisée en ce que** le colorant est sélectionné parmi acid blue 40, acid green 27, amido black, chicago sky blue, cibacron blue F3G-A, cibacron blue F-R, cibacron blue FN-R, cibacron blue P-3R, cibacron brillant blue H-GR, evans blue, fluorescéine, carminc d'indigo, tétrasulfonate d'indigo, isosulfan blue, bleu de méthylène, monastral blue, bleu patenté V, rhodamine et bleu de trypan.

8. Utilisation selon l'une des revendications précédentes, **caractérisée en ce que** le colorant est un colorant à fluorescence, émettant une lumière dans la plage de 380 à 780 nm de longueur d'ondes en cas d'irradiation aux UV ou à la lumière VIS.

9. Utilisation selon l'une des revendications précédentes, **caractérisée en ce que** le produit de contraste se présente sous forme stérile, non pyrogène et liophilisée.

10. Utilisation selon l'une des revendications 1 à 8, **caractérisée en ce que** le produit de contraste se présente sous forme de suspensions de particules dans un solvant aqueux, pharmaceutiquement acceptable, non pyrogène, stérile.

11. Utilisation selon l'une des revendications précédentes, pour visualiser le ou les ganglions lymphatiques sentinelles dans le cas d'un mélanome, d'un cancer du sein, du col de l'utérus ou de la prostate.

12. Nécessaire, comprenant un premier récipient avec le produit de contraste non radiomarqué, comprenant, en tant que composant essentiel, des particules de dextrane physiologiquement compatibles et pharmaceutiquement sans risque, présentant un poids moléculaire dans la fourchette de 1 à 5 millions et, pour jusqu'à 80 % ou plus, rapporté au nombre de particules, une grosseur de particule dans la fourchette de 30 à 300 nm, et ayant une couleur se distinguant à l'oeil nu de la couleur des tissus humains, et un deuxième récipient comprenant une solution de chlorure d'étain (II).

13. Produit de contraste marqué de manière radioactive, obtenu par mélange d'un produite de contraste non radiomarqué, comprenant, en tant que composant essentiel, des particules de dextrane physiologiquement compatibles et pharmaceutiquement sans risque, présentant un poids moléculaire dans la fourchette de 1 à 5 millions et, pour jusqu'à 80 % ou plus, rapporté au nombre de particules, une grosseur de particule dans la fourchette de 30 à 300 nm, et ayant une couleur se distinguant à l'oeil nu de la couleur des tissus humains, avec une solution de chlorure d'étain (II), neutralisation et conversion avec du ^{99m}Tc-pertechnétate.
